Europäisches Patentamt

European Patent Office

Office européen des brevets

(19)

(11) Veröffentlichungsnummer: **0 218 009**
**B1**

(12) ## EUROPÄISCHE PATENTSCHRIFT

(45) Veröffentlichungstag der Patentschrift:
**18.04.90**

(51) Int. Cl.⁴: **A61N 1/36**

(21) Anmeldenummer: **86107950.7**

(22) Anmeldetag: **11.06.86**

(54) **Herzschrittmacher.**

(30) Priorität: **04.10.85 DE 3535504**

(43) Veröffentlichungstag der Anmeldung:
**15.04.87 Patentblatt 87/16**

(45) Bekanntmachung des Hinweises auf die Patenterteilung:
**18.04.90 Patentblatt 90/16**

(84) Benannte Vertragsstaaten:
**DE FR GB IT NL SE**

(56) Entgegenhaltungen:
**FR-A- 2 032 996**
**FR-A- 2 109 073**
**US-A- 2 368 207**
**US-A- 3 358 690**

(73) Patentinhaber: **Siemens Aktiengesellschaft,
Wittelsbacherplatz 2, D-8000 München 2(DE)**

(84) Benannte Vertragsstaaten: **DE FR GB IT NL**

(73) Patentinhaber: **Siemens Elema AB, Röntgenvägen 2,
S-171 95 Solna 1(SE)**

(84) Benannte Vertragsstaaten: **SE**

(72) Erfinder: **Lekholm, Anders, Dr., Gnejsvägen 4,
S-161 39 Bromma(SE)**

ACTORUM AG

## Beschreibung

Die Erfindung betrifft einen Herzschrittmacher gemäß dem ersten Teil des Patentanspruchs 1.

Bei einem konventionellen Herzschrittmacher sollen die emittierten Stimulationsimpulse mit der natürlichen Herzaktivität übereinstimmen, damit sich der Herzschrittmacher und das Herz gegenseitig nicht stören. Dies wird bei konventionellen implantierbaren oder externen Herzschrittmachern dadurch erreicht, daß ein Detektor für QRS-Signale, P-Wellen und andere elektrische Herzsignale vorhanden ist. Diese elektrischen Signale werden über einen Verstärker in Verbindung mit geeigneten Filtern detektiert.

Durch die US-A-3 358 690 ist ein Herzstimulator bekannt, bei dem der Druck im Vorhof über einen Drucksensor abgefühlt wird, der auf Druckwechsel von niedrig nach hoch anspricht und dann über einen Verzögerungskreis und einen Schaltkreis sowie zwei Gleichspannungsquellen einen Stimulationsimpuls erzeugt. Es handelt sich also hier nicht um einen heute üblichen Herzschrittmacher mit einem Pulsgenerator für die Stimulation des Herzens, dessen Frequenz und Amplitude den jeweiligen Erfordernissen optimal angepaßt wird.

Der Erfindung liegt die Aufgabe zugrunde, einen Herzschrittmacher gemäß dem ersten Teil des Patentanspruchs, also mit einem steuerbaren Pulsgenerator, so auszubilden, daß auf die Erfassung der elektrischen Signale des Herzens zur Steuerung des Herzschrittmachers verzichtet werden kann.

Diese Aufgabe ist erfindungsgemäß gelöst durch den zweiten Teil des Patentanspruchs 1. Der Detektor kann entweder an einer separaten Leitung, in der Herzschrittmacherleitung, die zu einer Elektrode führt oder innerhalb oder außerhalb des Pulsgenerators selbst angeordnet sein. Durch geeignete Verstärkung und Signalverarbeitung, die analog und/oder digital sein kann, können P-Wellen, QRS-Komplexe und andere Herzsignale identifiziert werden.

Exit-Blocks, die elektrisch sehr schwer meßbar sind, Tachykardien und Fibrillationen können ebenfalls detektiert werden. Dies erfolgt bei Exit-Blocks über die fehlenden Herzgeräusche, bei Tachykardien über die Herzfrequenz. Im Falle einer ventrikulären Fibrillation besteht eine alternative Methode der Detektion in der Erfassung der Änderung des Herzgeräusches, d.h., Änderungen im Amplituden-Frequenzspektrum der detektierten akustischen Signale.

Das durch den Detektor erzeugte Signal wird für eine automatische Einstellung der Stimulationsimpulse verwendet, wodurch die Stimulationsschwelle bei vorprogrammierten Intervallen überwacht werden kann und die Stimulationsimpulse so eingestellt werden können, daß ein Exit-Block vermieden wird. Dies kann durch eine Überwachungsschaltung für die Herzkontraktion bei einem Stimulationsimpuls erfolgen. Eine solche Überwachungsschaltung kann auch dazu herangezogen werden, zu überwachen, ob ein Stimulationsimpuls auch eine Herzkontraktion hervorruft. Falls dies nicht der Fall ist, werden die Stimulationsimpulse vergrößert. Die gemessenen Werte können auch gespeichert werden.

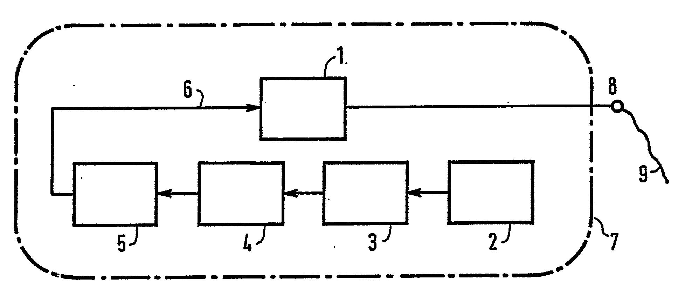

Die Erfindung ist nachfolgend anhand eines in der Zeichnung dargestellten Ausführungsbeispieles näher erläutert.

In der Zeichnung ist eine Schrittmacherelektronik 1 dargestellt, die an einer Ausgangsklemme 8 Stimulationsimpulse erzeugt, die über eine Leitung 9 der Stimulationselektrode des Herzschrittmachers zugeführt werden. Die Steuerung der Herzschrittmacherelektronik 1 erfolgt bei dem dargestellten Beispiel über ein Mikrofon 2 zur Erfassung der Herzgeräusche, dem ein Verstärker 3, eine Signalverarbeitungsstufe 4 und eine Detektorschaltung 5 nachgeschaltet sind. Demgemäß liegt am Eingang 6 der Herzschrittmacherelektronik 1 ein Steuersignal, das von der Kontraktion des Herzens abhängt. Sämtliche Komponenten 1 bis 5 können in dem Gehäuse 7 des Herzschrittmachers untergebracht sein.

Anstelle eines Mikrofons kann auch ein anderer geeigneter Detektor für die Herzgeräusche verwendet werden, der, wie ebenfalls eingangs geschildert, angeordnet werden kann. Eine Steuerschaltung für die Stimulationsimpulse zur automatischen Einstellung der Amplitude für die Überwachung der Reizschwelle und zur Verhütung eines Exit-Blocks, kann in der Herzschrittmacherelektronik 1 vorhanden sein. Diese Elektronik 1 kann auch eine Überwachungsschaltung für die Herzkontraktion bei einem Stimulationsimpuls enthalten.

## Patentansprüche

1. Herzschrittmacher mit einem Pulsgenerator (1), der durch Signale des Herzens gesteuert ist, **dadurch gekennzeichnet**, daß zur Erzeugung der Steuersignale ein Detektor (2) für die Herzgeräusche vorhanden ist, und daß der Detektor (2) an einer Steuerschaltung (3, 4, 5) für den Pulsgenerator (1) zur automatischen Einstellung der Stimulationsimpulse angeschlossen ist.

2. Herzschrittmacher nach Anspruch 1, **dadurch gekennzeichnet**, daß der Detektor ein Mikrofon (2) ist.

3. Herzschrittmacher nach Anspruch 1, **dadurch gekennzeichnet**, daß der Detektor ein Druckdetektor ist.

4. Herzschrittmacher nach Anspruch 1, **dadurch gekennzeichnet**, daß der Detektor ein Accelerometer ist.

5. Herzschrittmacher nach einem der Ansprüche 1 bis 4, **dadurch gekennzeichnet**, daß der Detektor (2) in einer Elektrodenzuleitung (9) des Herzschrittmachers angeordnet ist.

6. Herzschrittmacher nach einem der Ansprüche 1 bis 4, **dadurch gekennzeichnet**, daß der Detektor (2) innerhalb oder außerhalb des Herzschrittmachergehäuses (7) angeordnet ist.

7. Herzschrittmacher nach einem der Ansprüche 1 bis 6, **gekennzeichnet durch** eine Überwachungsschaltung (1) für die Herzkontraktion bei einem Stimulationsimpuls.

8. Herzschrittmacher nach einem der Ansprüche 1 bis 6, **gekennzeichnet durch** eine Überwachungsschaltung (1) für Tachykardien.

9. Herzschrittmacher nach einem der Ansprüche 1 bis 6, **gekennzeichnet durch** eine Überwachungsschaltung (1) für Fibrillationen.

**Claims**

1. Heart pacemaker with a pulse generator (1), which is controlled by signals from the heart, characterized in that a detector (2) for heart sounds is present for the production of the control signals, and in that the detector (2) is connected to a control circuit (3, 4, 5) for the pulse generator (1) for the automatic adjustment of the stimulation impulses.

2. Heart pacemaker according to claim 1, characterized in that the detector is a microphone (2).

3. Heart pacemaker according to claim 1, characterized in that the detector is a pressure detector.

4. Heart pacemaker according to claim 1, characterized in that the detector is an accelerometer.

5. Heart pacemaker according to one of claims 1 to 4, characterized in that the detector (2) is arranged in an electrode lead (9) of the heart pacemaker.

6. Heart pacemaker according to one of claims 1 to 4, characterized in that the detector (2) is arranged inside or outside the heart pacemaker housing (7).

7. Heart pacemaker according to one of claims 1 to 6, characterized by a monitoring circuit (1) for heart contraction during a stimulation impulse.

8. Heart pacemaker according to one of claims 1 to 6, characterized by a monitoring circuit (1) for tachycardia.

9. Heart pacemaker according to one of claims 1 to 6, characterized by a monitoring circuit (1) for fibrillations.

**Revendications**

1. Stimulateur cardiaque comportant un générateur d'impulsions (1) commandé par les signaux du cœur, caractérisé par le fait que pour l'obtention des signaux de commande, il est prévu un détecteur (2) servant à détecter les bruits cardiaques et que le détecteur (2) est raccordé à un circuit (3, 4, 5) de commande du générateur d'impulsions (1), de manière à régler automatiquement les impulsions de stimulation.

2. Stimulateur cardiaque suivant la revendication 1, caractérisé en ce que le détecteur est un microphone (2).

3. Stimulateur cardiaque suivant la revendication 1, caractérisé par le fait que le détecteur est un capteur de pression.

4. Stimulateur cardiaque suivant la revendication 1, caractérisé par le fait que le détecteur est un accéléromètre.

5. Stimulateur cardiaque suivant l'une des revendications 1 à 4, caractérisé par le fait que le détecteur (2) est disposé dans un conducteur (9) relié à une électrode du stimulateur cardiaque.

6. Stimulateur cardiaque suivant l'une des revendications 1 à 4, caractérisé par le fait que le détecteur (2) est disposé à l'intérieur ou à l'extérieur du boîtier (7) du stimulateur cardiaque.

7. Stimulateur cardiaque suivant l'une des revendications 1 à 6, caractérisé par un circuit (1) servant à contrôler la contraction du cœur lors de l'applica-tion d'une impulsion de stimulation.

8. Stimulateur cardiaque suivant l'une des revendications 1 à 6, caractérisé par un circuit (1) servant à contrôler des tachycardies.

9. Stimulateur cardiaque suivant l'une des revendications 1 à 6, caractérisé par un circuit (1) servant à contrôler des fibrillations.